(19) Europäisches Patentamt / European Patent Office / Office européen des brevets

(11) **EP 2 292 142 B1**

(12) **EUROPEAN PATENT SPECIFICATION**

(45) Date of publication and mention
of the grant of the patent:
**19.09.2012 Bulletin 2012/38**

(51) Int Cl.:
**A61B 5/026** (2006.01)

(21) Application number: **10169669.8**

(22) Date of filing: **15.07.2010**

(54) **Method and device for evaluation of blood fluidity**

Verfahren und Vorrichtung zur Bestimmung der Blutfluidität

Méthode et dispositif pour l'évaluation de sang fluidité

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **04.09.2009 JP 2009205080**

(43) Date of publication of application:
**09.03.2011 Bulletin 2011/10**

(73) Proprietor: **Adtex Inc.
Gunma 370-1201 (JP)**

(72) Inventors:
• **Koshino, Shinji**
**Takasaki-shi Gunma 370-1201 (JP)**
• **Shimizu, Hiroki**
**Takasaki-shi Gunma 370-1201 (JP)**
• **Sato, Hiroo**
**Takasaki-shi Gunma 370-1201 (JP)**

(74) Representative: **Berkkam, Ayfer
AZe Marka Patent Ltd.Co.
Becker-Gundahl-Strasse 49
81479 Munich (DE)**

(56) References cited:
EP-A1- 1 623 668      WO-A1-2004/073514
JP-A- 2008 183 257      US-B1- 6 178 342

**Description**

FIELD OF THE INVENTION

The present invention relates to a method and a device that can evaluate blood fluidity (principally, blood viscosity) from outside the body of a subject in a simple manner.

BACKGROUND OF THE INVENTION

Recently the demand for knowledge of blood viscosity has become strong, because it is a datum that is extremely useful for the maintenance of health. However generally, in order to know the viscosity of the blood of a subject, it has been necessary to perform sampling of his blood for subsequent testing, and there has not been any convenient and non-invasive method for determining blood viscosity.

It has been attempted to evaluate heart function, blood pressure, the state of hardening of the arteries, and so on by objectively measuring the state of the peripheral circulation within the body of a living subject, although in the prior art this has not been done in order to detect blood viscosity. The type of device included in Patent Document (JP11-089808) includes a pressure device that presses upon a predetermined site upon the body of a subject in order to empty that site of blood, a blood return amount detection device that optically detects the amount of blood returning to this predetermined site upon the body of the subject when the pressing by the pressure device is removed, and a blood return amount display means that displays the change over time of the amount of return blood detected by the blood return amount detection device as a graph along a predetermined time axis; and it is arranged to perform evaluation of various functions according to the speed of return of blood flow during return from the blood empty state.

EP-A-1 623 668 discloses a method comprising the features of the preamble of claim 1, and a device comprising the features of the preamble of claim 5.

SUMMARY OF THE INVENTION

With a technique such as that described in Patent Document (JP11-089808), during this measurement of the change of blood flow when pressure is removed, since the blood starts to fill the entire circulatory system at once when the pressure is removed, accordingly, even if for example the wavelength of the light or the gap between the optical elements is optimized, it is still difficult to distinguish perfectly between the arterial system and the capillary system. Furthermore, a so-called blood pressure influence is exerted by the pressure of the blood that is being squeezed out from the heart, and it is difficult to perform calibration, since the blood pressure changes moment by moment. Moreover the blood pressure pulsates in correspondence to the beating action of the heart, and, while it is necessary to eliminate this pulsation component, this elimination is difficult. Thus since, irrespective of the fact that it is only actually desired to obtain blood flow data relating to the capillary system for evaluation of the blood viscosity, nevertheless a lot of superfluous information relating to the arterial system and so on is undesirably included in the measurement data, accordingly it has been impracticable to perform blood viscosity evaluation with good sensitivity.

The present invention has been conceived in the light of the above situation, and its object is to provide a method and a device that can evaluate the fluidity of the blood of a subject in a simple manner with good sensitivity.

The invention is defined in the appended claims.

According to the present invention, a method and device are supplied which can conveniently evaluate blood fluidity. And since, according to the present invention, the light emission element and the light reception element may be installed upon the pressure member, accordingly it is possible to reduce the dimensions of the pressure member. Since, if the dimensions of the pressure member are small, the test site region upon the skin of the subject against which the pressure member is pressed is also small, accordingly it becomes possible to press in a pin-point manner against a measurement region in the test site, so that it is possible to alleviate measurement errors that might occur due to compression of the peripheral region around the measurement region.

And, according to the present invention, blood fluidity evaluation is performed in synchronization with measurement of the pulse of the subject, so that it is also possible to alleviate errors due to the pulse of the subject. And, in yet another embodiment, it is also possible to consider optimization of the relative position of the test site and the pressure member by utilizing measurement of the pulse before evaluation of the blood fluidity, and thereby a further contribution may be made to enhancing the accuracy during the blood fluidity evaluation.

BRIEF DESCRIPTION OF THE DRAWINGS

Fig. 1 is a block diagram showing the general structure of a blood fluidity evaluation device;
Fig. 2 is a schematic figure showing measurement by a device according to the present invention;
Fig. 3 is a figure showing a model case of measurement data for evaluation of blood fluidity;
Fig. 4 is a figure showing another model case of measurement data for evaluation of blood fluidity;
Fig. 5 is a figure showing an arterial model when the change of flow is slow;
Fig. 6 is a figure showing an equivalent circuit for a venous model;
Fig. 7 is a figure showing another equivalent circuit for a venous model;
Fig. 8 is a figure showing an equivalent circuit for a capillary system model;
Fig. 9 is a figure showing the physical meaning of a differential equation for a capillary system model;
Fig. 10 is a figure showing blood amount Q and observed light reception intensity $I_R$;
Fig. 11 is a figure schematically showing the meaning of an evaluation parameter;
Fig. 12 is a figure for explaining light reception intensity when light propagates through a plurality of layers;
Fig. 13 is a figure showing a way in which hemoglobin density changes after application of pressure;
Fig. 14 is a figure showing the flow of processing;
Fig. 15 is a figure showing the physical meaning of a quantity $(t_2-t_1)$;
Fig. 16 is a figure showing a model case of measurement data; and
Fig. 17 is a figure showing another model case of measurement data.

In the figures, the reference symbols mean as follows:

1:        blood fluidity evaluation device
10:       pressure member
11:       light emission element
12:       light reception element
21:       pressure device
22:       pressure device drive circuit
23:       light emission element drive circuit
24:       received optical signal detection circuit
25:       CPU
26:       operation switch
27:       display device
30:       unit for evaluating blood fluidity
31:       unit for evaluating pulse measurement
100:      test site
110:      pulse measurement site

The present invention will now be described in detail with reference to the drawings. However, the present invention is not to be considered as being limited to the embodiments disclosed or shown in the figures.

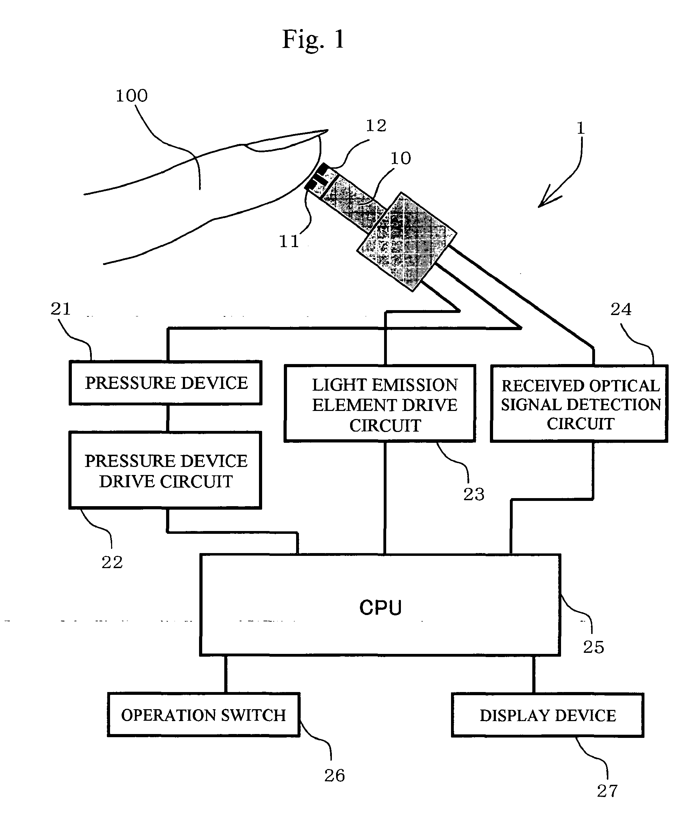

Fig. 1 is a block diagram showing the general structure of a blood fluidity evaluation device.

This blood fluidity evaluation device 1 comprises a pressure member 10 that is pressed against a test site 100 upon a test subject, such as a finger tip or the like. This pressure member 10 can be pressed against, or withdrawn from, the test site 100 upon the test subject under the control of a drive control device comprising a pressure device 21 and a pressure device drive circuit 22 and so on that will be described hereinafter. A light emission element 11 and a light reception element 12 are installed upon the pressure member 10. The light emission element 11 is an element that illuminates light to irradiate the test site 100 against which it is pressed. The light emitted from this light emission element 11 is scattered within the test site 100, and is then detected by the light reception element 12. As will be described hereinafter, it is possible to calculate the amount of blood in the test site 100 from the correlation between the light emission from the light emission element 11 and the measurement data received from the light reception element 12. And, from data such as the degree and the duration of application of pressure by the pressure member 10 and the change over time of this blood amount and so on, it is possible to evaluate the fluidity of the blood of the subject at the test site 100. This blood fluidity evaluation device 1 is provided with a calculation means that calculates the blood amount and the blood fluidity in this manner, and is also provided with a display means that displays at least one of these quantities.

The pressure member 10 is built so that, each time it moves forwards and backwards along its own axis (for example), it is pressed against the test site 100, which is covered with the skin of the subject or the like. This movement of the pressure member 10 is controlled by the pressure device 21, the pressure device drive circuit 22 and so on via electrical control of an actuator or the like. In the embodiment shown in the figures, the drive control device comprises the pressure device 21 and the pressure device drive circuit 22.

In order for the pressure member 10 to be pressed in a predetermined position and orientation against the test site 100, it is desirable to prepare in advance a predetermined measurement stand (not shown in the drawings), upon which the test site (the finger of the subject or the like) is set. At this time, in order to reduce the influence upon blood flow to a minimum level, it is desirable for the measurement stand to be built so as not to exert any compression upon the test site 100. The positional determination of the pressure member 10 in relation to the test site 100 at high accuracy will be described hereinafter.

The light emission element 11 is installed to the pressure member 10. The driving of this light emission element 11 is controlled by a light emission element drive circuit 23, while the signal from the light reception element 12 is inputted via a received optical signal detection circuit 24 to a CPU 25, after having been amplified by an amplification circuit or the like (not shown in the drawings), according to requirements. It should be understood that the number of light emission elements 11 and the number of light reception elements 12 are not to be considered as being particularly limited. For example, it would be acceptable to provide a plurality of light reception elements 12 and to perform calculation to evaluate the blood viscosity by performing averaging processing upon the plurality of received optical signals from these light reception elements 12. By doing this, it is possible to mitigate the influence of errors due to the position of measurement and so on. The light emission element 11 is adapted to irradiate light against the surface of the test site 100, such as the skin of the finger of the subject or the like, and the light reception element 12 is adapted to receive the light returning from the test site 100.

It is desirable for the light emission element 11 to be adapted to emit light of a short wavelength such as blue light or light close to blue, and for the light reception element 12 to be of a type that is capable of receiving that kind of light.

In the embodiment of Fig. 1, the CPU 25 calculates the amount of blood from the measurement data received by the light reception element 11. The blood fluidity (principally, its viscosity) is also calculated by the CPU 25. The blood fluidity is obtained from the change over time of the amount of blood from the moment the pressure member 10 starts to exert pressure upon the test site. The procedure by which this calculation is performed will be described hereinafter. Furthermore, the CPU 25 controls the operation of the light emission element drive circuit 23, of the received optical signal detection circuit 24, of a blood viscosity calculation circuit (not shown in the drawings), and of a display device 27. Yet further, the CPU 25 receives operation signals from an operation switch 26.

Next, the combined use as a pulse measurement means will be explained.

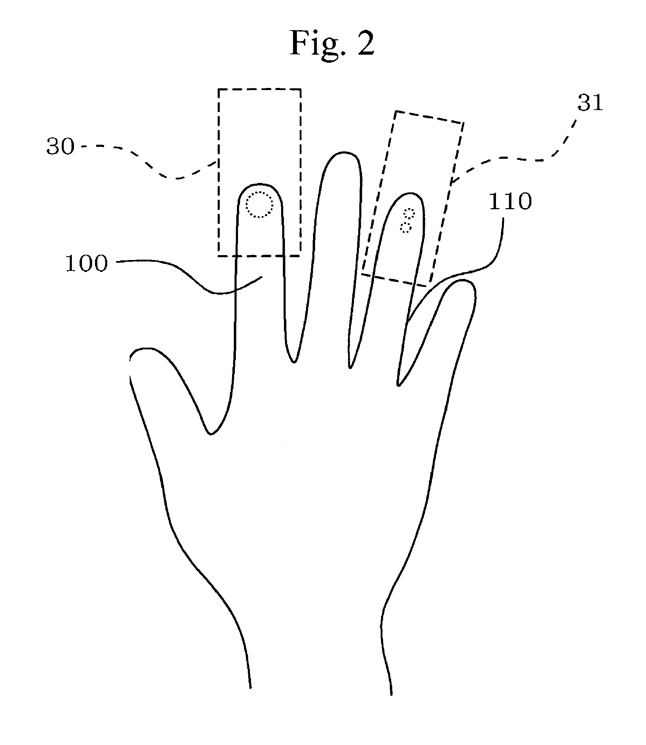

Fig. 2 is a schematic figure showing measurement by a device according to the present invention. In the case of this embodiment shown in the figure, there are provided a unit 30 for evaluating the blood fluidity at the test site 100, which is located at the tip of the index finger of the subject, and a unit 31 for measurement of the pulse in his third finger. In this case, the index finger of the subject is taken as being the test site 100, and his third finger is taken as being the pulse measurement site 110. The unit 30 comprises a pressure member, a light emission element, and a light reception element as described above. And the unit 30 and the unit 31 can be operated together by a calculation means as described above (not shown in the drawing), as will be described hereinafter.

The concrete structure of the pulse measurement means is not particularly limited. As one example, it may be suggested that a unit 31 similar to the unit 30 and having a pressure member 10, a light emission element 11, and a light reception element 12 may be provided. The way in which the pulse of the subject is measured by this unit is as described below. It is possible to detect the pulse waves by irradiating light upon the pulse measurement site 110 while pushing against it with a weak force, and by detecting the light scattered back. The pressing force during detection of the pulse waves should be a weak pressure of a level that does not easily cause the blood at the pressing site substantially to flow out to its periphery, and, in concrete terms, is desirably around 30 to 60 mmHg. A pulse measurement means that utilizes this type of pulse wave detection may be employed with the present invention.

As described above, since the detection of the pulse waves can be performed by the use of light in a similar manner to the detection of the blood amount, accordingly, during the detection of the blood amount with the unit 30, there is a possibility that an error may be introduced due to the pulse waves. For example the start of pressing by the pressure member 10 can cause error due to the pulse in the tissue at the test site 100, since, at high accuracy, the amount of blood in the tissues is slightly different directly after the blood has flowed in and directly before it flows in. In order to eliminate or reduce this error, in accordance with the present invention, it is proposed to employ the pulse measurement data at the pulse measurement site 110 during calculation of the blood fluidity, and thereby to reduce the calculation error due to the pulse of the subject.

Furthermore, the operation of pressing by the unit 30 upon the site 100 for evaluation of the blood fluidity is controlled while employing the pulse measurement data at the pulse measurement site 110. For example, a method may be suggested of observing the pulse waves with the unit 31 as it measures the pulse of the subject, and starting the pressure upon the test site 100 according to the time point that the flow of blood into the test site 100 reaches its maximum, or according to the time point that it reaches its minimum, or the like. In this case, it would be acceptable to measure the pulse with a unit 31 that is different from the unit 30 for evaluating the blood fluidity, as shown in Fig. 2; or, alternatively, it would be acceptable first to measure the pulse with the same unit as the unit for blood fluidity evaluation, and then to change over to measurement of the blood fluidity at a predetermined moment. However, from the point of view of not applying any force to the test site 100 before evaluating the blood fluidity, it is desirable to measure the pulse by providing a unit 31 which is separate from the unit 30 that is used for evaluation of the blood fluidity.

Next, concepts for enhancing the accuracy of determination of the position of the test site by taking advantage of the pulse measurement will be explained.

Before the measurement of the blood fluidity, the pressure member 10 is pressed by the unit 30 for evaluation of blood fluidity against the test site 100 with the weak force described above, and pulse measurement data is acquired by absorption of light. At this time, the detected pulse wave component may become weak if the positional relationship between the pressure member 10 and the test site 100 is not appropriate. Accordingly measurement data for the pulse may be measured while shifting the relative position between the pressure member 10 and the test site 100, and a relative position at which this measurement data reaches a local maximum may be considered as being a suitable position for blood fluidity evaluation. If the index finger of human being is taken as the test site, then, normally, it is possible to determine a suitable measurement position by obtaining the position within a region of 5 mm around the finger tip at which the pulse measurement data reaches a local maximum.

Next, the theory of blood viscosity measurement will be discussed.

Capillaries are located at shallow positions under the skin, while arteries and veins are located at deep positions. When compression force is applied to the skin, while the blood flows out rapidly from the arteries and veins to other portions since they are large diameter flow conduits, by contrast, the blood flows slowly out from the capillaries to peripheral regions because they are small diameter flow conduits whose flow resistances are great. Moreover, since if the capillaries are compressed their blood flow passages are further squeezed down, accordingly the amount of outflow comes to be further reduced by the influence of changes of viscosity such as agglomeration or reduction in deformability of the red blood cells.

The change over time (i.e. the speed) of this flowing out of blood depends upon the resistance to blood flow (of which the viscosity of the blood is one element). Thus in this embodiment the viscosity, which is one element of the resistance to blood flow, may be estimated by optically detecting the change over time of the blood outflow from within the capillaries.

Generally visible light is largely absorbed by hemoglobin within a living organism or by other structural substances of the organism, and can hardly pass through the tissues at all. However, when blood flows out from within the capillaries due to application of pressure to the skin, the amount of blood (i.e. of hemoglobin) drops along therewith, and the absorption of light within the body of the organism decreases as compared to its value before the application of pressure. In other words, some of the blood within the capillaries flows out due to the application of pressure, so that, along with this reduction of the amount of blood (i.e. of the amount of hemoglobin) more of the light can pass through.

Furthermore, light of short wavelength almost does not pass through the body of a living organism at all. In other words, observation of light of short wavelength that has been irradiated against a living body is considered to be observation of light that has arrived by propagation through the capillaries directly under the skin. It becomes possible, by taking advantage of this theory, to observe the mode of blood outflow from within the capillaries due to the application of pressure, and from this it becomes possible to estimate the change of density of hemoglobin in the tissues.

In this case, it is desirable for the light emission element 11 and the light reception element 12 to be set so as only to detect the amount of blood in the capillaries under the skin, and not to detect the amount of blood in the arteries or in the arterioles. For this, it is desirable to position the light emission element 11 and the light reception element 12 close together, so that they can detect the blood flow in the shallow portions of the tissue with high sensitivity. Moreover, while the light that is irradiated upon the skin may be long wavelength light such as red or infra-red light, it is more desirable for it to be short wavelength light such as blue light or light close to blue.

This is because, the longer is the wavelength of the light, the less it is scattered enroute, so that the light can enter to deep portions under the skin of the subject (and can reach the arteries); while, the shorter is the wavelength of the light, the more it comes to be returned from shallow positions under the skin, and does not reach deep portions (i.e. does not reach the arteries). In other words, while the light emitted from the light emission element 11 enters into the skin and is repeatedly scattered within the skin, and then a portion thereof is returned to the surface of the skin, since blood vessels are present upon the path of this light, the returning light experiences absorption due to this blood. Accordingly light of short wavelength is employed, in order for the light not to experience any influence due to absorption by blood within the arterioles or the arteries which are at deep portions under the skin, but rather only to experience the action of light absorption by blood within the capillaries which are at shallow portions under the skin, with the light then being returned to the surface of the skin and emerging therefrom.

For example, if a blue color LED is used as the light emission element 11, then desirably the photo-transistor used for the light reception element 12 should be positioned within a distance of around 3 mm therefrom.

Next, the method of measurement will be explained.

During measurement, the pressure member 10 is approached to the vicinity of the test site 100 such as a finger tip of the subject or the like. Desirably, a measurement stand (not shown in the drawings) that is molded so as to be capable of fixating the test site 100 is employed. Then the operation control device comprising the pressure device 21 and the pressure device drive circuit 22 is operated by actuation of the operation switch 26 so that the pressure member 10 moves forward so as to press against the test site 100, and this pressing is continued with a predetermined pressing force.

In this state, optical measurement by the light emission element 11 and the light reception element 12 installed in the pressure member 10 is performed. And, as time elapses, the signal from the light reception element 12 is amplified and is inputted to a blood viscosity calculation circuit (i.e. to the CPU 25), which calculates data that specifies the nature of the blood flow in the capillaries (i.e. the resistance to the flow of blood) from the time rate of change of the value of the received light signal (i.e. from the light reception intensity). This data includes an indicator that specifies the fluidity or the viscosity of the blood within the capillaries, and this indication is displayed by the display device 27.

For example, theoretically, the greater is the amount of blood, the more light is absorbed, so that the smaller is the amount of received light. And the converse is true: the smaller is the amount of blood, the greater is the amount of received light. Accordingly, upon further consideration, the fact that the amount of received light is great means that the absorption experienced by the light is small, that the amount of blood is low, and that it has been easy for the blood to be displaced by pressure, in other words means that the viscosity of the blood is low; while the fact that the amount of received light is small means the converse.

With the present invention, evaluation of the resistance to blood flow is performed on the basis of the change over time of the blood outflow from the capillaries when the surface of the test site 100 is pressed. Influence is not exerted upon the blood flow in arteries and veins and so on that are at deeper positions than the capillaries, so that it is possible to evaluate the fluidity of the blood (principally, the viscosity of the blood) with good sensitivity. In other words, since in the first place the blood vessel resistance of the arteries and the veins is low as compared with that of the capillaries, and the blood therein flows out rapidly when pressed, accordingly these do not exert any substantial influence upon this measurement, and it is possible to measure only the outflow of blood remaining in the capillary system. And, on the basis of this measurement, it is possible to evaluate the fluidity of the blood (principally its viscosity) with good accuracy. In particular it is considered that, by measuring the change of the amount of blood remaining under the skin of the subject optically from the exterior by taking advantage of absorption of light by hemoglobin, it is possible to make the measurement setup simpler.

It is desirable for the force exerted while pressing upon the skin to be a higher pressure than the maximum blood pressure within the human body. This is because it is necessary partially to stop the circulation of blood that takes place naturally within the body, and to eject the blood in the blood vessels in the portion upon which pressure is exerted to the periphery of that portion. However, since the arteries that are present in the finger tips or the ear lobes or the like (it is also possible to perform measurement upon the ear lobes, rather than the finger tips) are not thick arteries but are merely arterioles, and since the blood pressure within the arterioles is lower than that within the arteries, accordingly it will also be acceptable for the applied pressure not to be necessarily higher than the maximum predicted blood pressure, and, in this case, it may be anticipated that the stress upon the test subject will be alleviated.

The time from the start of exertion of pressure over which the flow of blood is observed may generally be no more than a few seconds. This is because the data obtained during this interval reflect changes in the fluidity of the blood very well.

Actually there is a possibility that errors in measurement may occur, since the density of capillaries and the density of blood vessels at the measurement site may vary according to the constitution of the test subject. As a means for reducing this possibility, it would be acceptable to provide a large number of light emission elements 11 arranged at almost regular spacings around the periphery of a single light reception element 12, and to average a number of simultaneous measurements over different areas of the skin of the subject by illuminating these light emission elements 11 one after another.

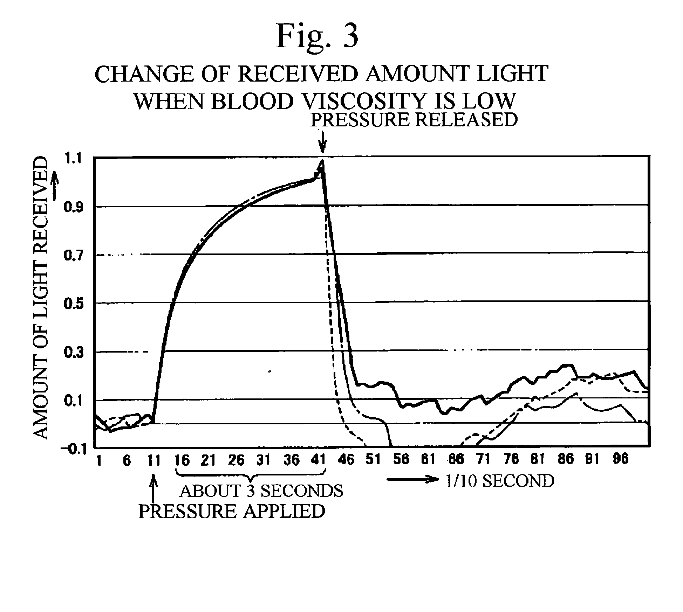

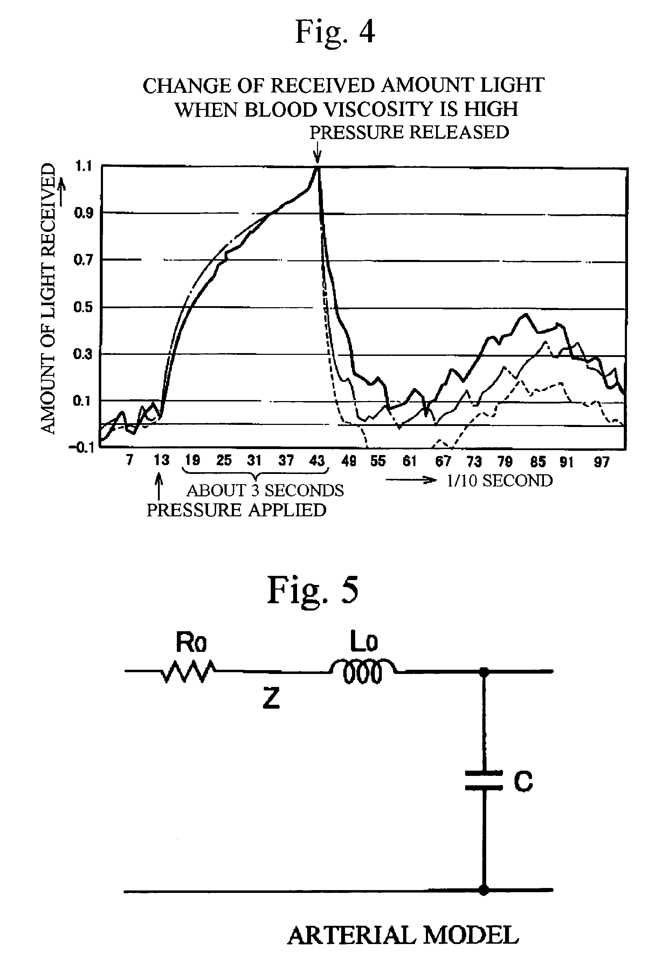

Figs. 3 and 4 show model cases of measurement data for evaluating blood fluidity. These figures show the changes in the amount of light received. With the measurement data of Fig. 3, the slope of the rising shoulder of the waveform of the amount of received light is gentle. On the other hand, with the measurement data of Fig. 4, the slope of the rising shoulder of the waveform of the amount of received light is abrupt. Due to this, when these two graphs are compared, the evaluation may be reached that in the example of Fig. 3 the blood viscosity is low, while in the example of Fig. 4 the blood viscosity is high.

As shown by the examples of Fig. 3 and Fig. 4, if a blood viscosity evaluation parameter $\alpha$ which corresponds to the amount of blood under the skin is calculated from the measured data for the amount of light received, then it is possible to evaluate the viscosity of the blood objectively on the basis of this parameter $\alpha$. It should be understood that this parameter $\alpha$ is a value that does not depend upon the total blood flow rate change in the capillary system due to arterial contraction; the method by which $\alpha$ is obtained will be explained below.

First, in order to obtain this parameter $\alpha$ for numerical evaluation of the blood viscosity, we initially try considering electrical circuit models for the blood vessel system. If the blood vessel system is modeled as an electrical circuit, then voltage V may be considered as corresponding to the internal blood vessel pressure P, while electrical current I corresponds to the blood flow rate S.

An arterial model that is applicable when the change of flow is slow is as shown in Fig. 5.

In this case, the resistance to blood flow (i.e. the blood vessel resistance) $R_0$ is given by Equation (1):

$$R_0 = \frac{8\mu}{\pi R^4} \qquad \left[\frac{Pa\,s}{m^4}\right] \quad \cdots (1)$$

And the blood reactance $L_0$ is given by Equation (2):

$$L_0 = \frac{4}{3}\frac{\rho}{\pi R^2} \qquad \cdots (2)$$

The capacitance C of the circulatory system (i.e. the amount of blood accumulated within the blood vessels) is given by Equation (3):

$$C = \frac{2\pi R^3(1-\sigma^2)}{Eh} \qquad \cdots (3)$$

Here:

$\rho$ is the mass of the blood;
$\mu$ is the coefficient of viscosity of the blood;
$\sigma$ is the Poisson ratio of the blood vessel walls;
R is the internal diameter of the blood vessels;
and E is the Young's modulus of the blood vessel walls.

Equation (1) that gives the resistance to blood flow $R_0$ shows that this resistance is the smaller, the larger is the radius R of the blood vessels, and that the resistance is the larger, the greater is the viscosity of the blood. Generally $R_0$ in Equation (1) is termed the blood vessel resistance, but this quantity depends upon the viscosity of the blood so that the blood viscosity $\mu$ is included in the equation, and here it will be termed the resistance to blood flow, from its precise meaning, which is the resistance that is experienced when the blood flows.

Next, the venous system will be considered.

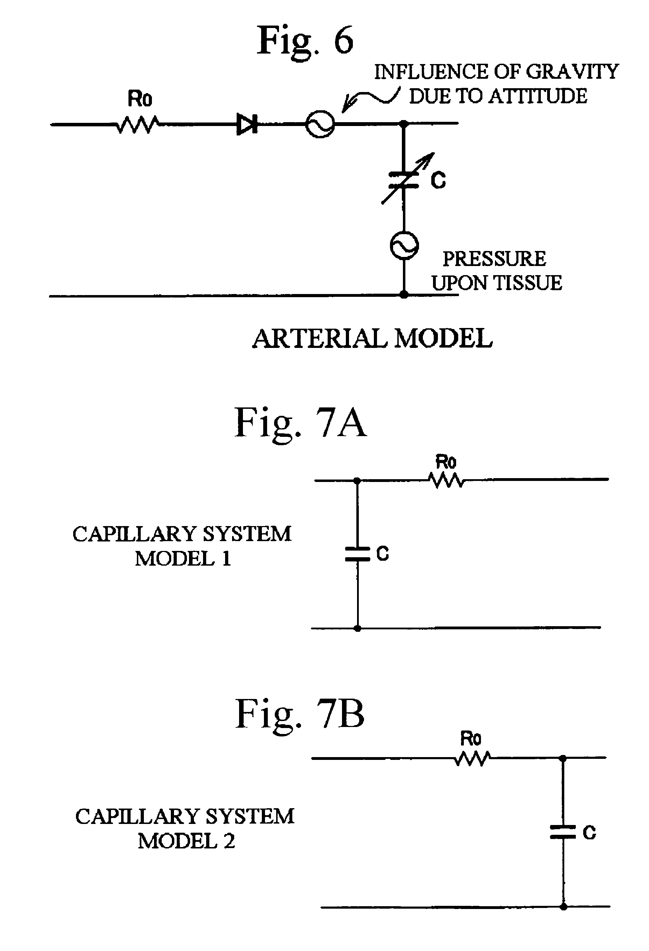

Since venous valves are present in veins through which steady flow passes in order to prevent reverse flow, an equivalent electrical circuit as shown in Fig. 6 may be obtained by modeling these venous valves with diodes. The influence of tissue pressure and the influence of gravity due to attitude are included as elements in this model. Here, $R_0$ is the resistance to blood flow (i.e. the blood vessel resistance), and is given by the following Equation (4):

$$R_0 = \frac{8\mu}{\pi R^4} \qquad \cdots (4)$$

Next, the capillary system will be considered.

Capillaries are blood vessels that are made from a single layer of endothelial cells, and they do not have muscle tissue that itself contracts, as arteries do. Moreover, since they also do not have venous valves that can be modeled as ideal diodes, as the venous

system does, equivalent circuits in which the venous model is changed as in Fig. 7 may be thought of as models of the capillary system. In this case, the two models #1 and #2 shown in Figs. 7(a) and 7(b) are respectively applicable for the period when blood flows out from the capillary system, and for the period when blood flows into it.

In Fig. 7, the resistance to blood flow (i.e. the blood vessel resistance) $R_0$ is the same as in the case of the venous system, and the following Equation (5) holds:

$$R_0 = \frac{8\mu}{\pi R^4} \qquad \left[\frac{Pa\,s}{m^4}\right] \qquad \cdot\cdot\cdot(5)$$

Here, using the blood vessel radius R, the amount of blood Q accumulated in the capillaries may be approximated by Equation 6:

$$Q = n\,\pi\,l_m\,R^2 \qquad [m^3] \qquad \cdot\cdot\cdot(6)$$

It should be understood that n is the basic number of capillaries being considered, and $l_m$ is the average length of these capillaries.

When Equation (6) is substituted into Equation (5), the following Equation (7) is obtained:

$$R_0 = \frac{8\pi n^2 l_m{}^2 \mu}{Q^2} \qquad \cdot\cdot\cdot(7)$$

Here, it is necessary to pay attention to the fact that the resistance to blood flow is proportional to the blood viscosity $\mu$, and also to the fact that the resistance to blood flow itself changes in dependence upon the total amount of blood that is accumulated in the blood vessels. In other words, if it is considered at what time the constant external pressure is applied to the blood vessels and the internal blood flows out into the venous system, the resistance to blood flow R increases according to the magnitude of the total blood amount Q that decreases along with the passage of time, and this process operates to reduce the amount of blood flowing out.

Next, the state of blood movement when pressure is applied will be considered.

Here, the case in which the applied pressure is a higher pressure than the highest blood pressure in any artery will be considered. At this time, considering the arteries, the internal blood that is passing along the arteries that have low blood resistance is squeezed out rapidly (since the radius of an artery is sufficiently great as compared to the radius of a blood vessel of the capillary system for its resistance to blood flow to be small). A similar phenomenon takes place in relation to the venous system, so that the internal blood that is passing along the venous system that has low blood flow resistance flows out rapidly along with the application of pressure.

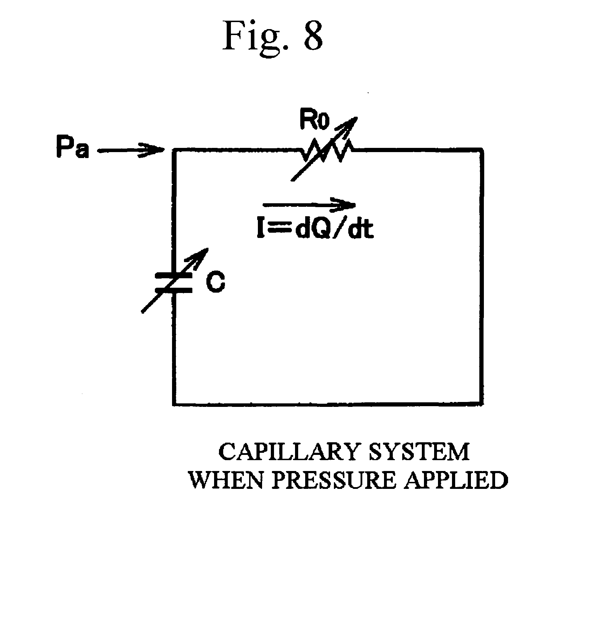

However, the blood that has accumulated in the capillaries is subjected to a pressure that is equal to the pressure applied from the exterior, and, while it tries to flow out into the venous system, this outflow proceeds slowly, due to the high resistance to blood flow of the capillary system. Furthermore, since the radius R of the capillary system decreases along with outflow of the blood in the capillaries, accordingly the resistance to blood flow becomes greater (refer to Equation (5) above), so that the outflow proceeds yet more slowly. At this time, since it may be considered that the resistance to blood flow of the venous system is sufficiently small as compared to that of the capillary system, accordingly the equivalent electrical circuit is as shown in Fig. 8.

Here:

C corresponds to the capacity within the capillaries (here the accumulated electric charge Q corresponds to the blood amount);
$R_0$ is the resistance to blood flow (refer to Equation (7));
and Pa is the pressure gradient applied from the exterior (units Pa/m).

The blood flow rate (electrical current) I that flows out to the exterior from this capillary system is given by Equation (8):

$$I = \frac{P_a}{R} = \frac{P_a}{8\pi n^2 l_m{}^2 \mu} Q^2 \qquad [m^3/s] \qquad \cdot\cdot\cdot(8)$$

Here, when I=dQ/dt is considered, it is possible to obtain Equation (9) as the differential equation satisfied by the amount of blood Q that is accumulated in the capillary system. In other words, this differential equation (9) that the amount of blood Q in the capillaries after the application of pressure must satisfy is obtained:

$$\frac{dQ}{dt} = -\alpha\, Q^2 \qquad \cdots (9)$$

However, $\alpha$ is given by Equation (10):

$$\alpha = \frac{P_a}{8\pi n^2 l_m^{\,2} \mu} \qquad \left[\frac{Pa/m}{m^2\, Pa\, s} = \frac{1}{m^3\, s}\right] \qquad \cdots (10)$$

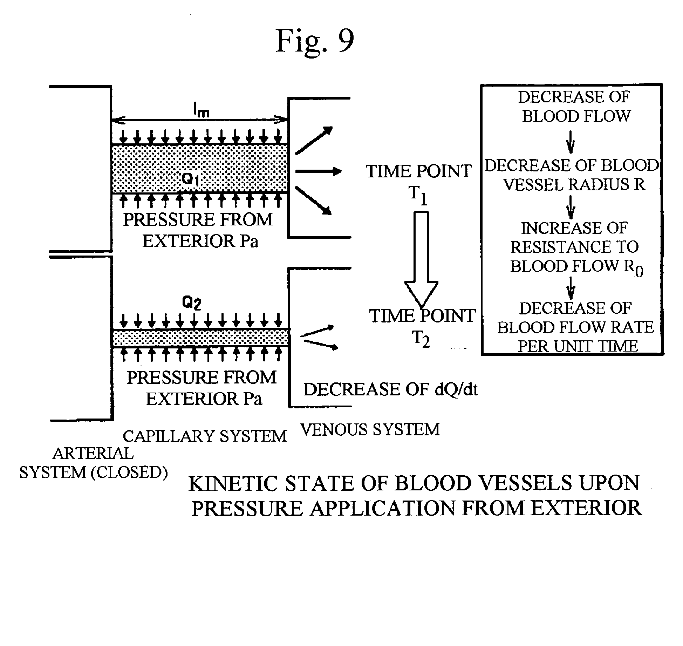

When the physical meaning of this differential equation is shown in a figure, we obtain Fig. 9. Fig. 9 shows that, when an external pressure of Pa is applied at the time point T1, outflow of blood in the blood vessel takes place due to this pressure, the radius R of the blood vessel decreases due thereto, increase of the resistance to blood flow R0 occurs due to this decrease of the blood vessel radius, and, as a result, decrease of the blood flow rate per unit time (dQ/dt) occurs.

Next, the relationship between the amount of blood Q in the capillaries and the light reception intensity will be considered.

The light intensity that is observed by an optical detector (the light reception element) again placed at the surface of the body, partway along a capillary, is given by Equation (11):

$$I_R = K\, A_B\, A_T\, I_T \qquad \cdots (11)$$

Here, $A_B$ and $A_T$ respectively denote the attenuation of light within the blood and the attenuation of light within the tissues. Moreover $I_T$ is the intensity of the incident light and K is a coefficient. When the change over time of Equation (11) is considered, the following Equation (12) is obtained:

$$\frac{\partial I_R}{\partial t} = K\, A_T\, I_T\, \frac{\partial A_B}{\partial t} \qquad \cdots (12)$$

When the fact is considered that the following relationship (13):

$$\frac{\partial A_B}{\partial t} = -\beta\, \frac{\partial Q}{\partial t} \qquad \cdots (13)$$

between the attenuation due to the blood and the amount of blood approximately holds, then, as the relationship between the light reception intensity and the amount of blood, the following Equation (14) is obtained:

$$\frac{\partial I_R}{\partial t} = -K\, \beta\, A_T\, I_T\, \frac{\partial Q}{\partial t} \qquad \cdots (14)$$

The differential equation (9) satisfied by the amount of blood Q in the above described capillary can be solved by analysis. First we divide both sides by $Q^2$, and, with Q' being written for the differential coefficient of Q, the following Equation (15) is obtained:

$$\frac{Q'}{Q^2} = -\alpha \qquad \cdots (15)$$

This Equation can be converted to the following Equation (16):

$$\left(\frac{1}{Q}\right)' = \alpha \quad \cdot \cdot \cdot (1\,6)$$

By doing this, the following Equation (17) is obtained:

$$Q = \frac{1}{\alpha t + C} \quad \cdot \cdot \cdot (1\,7)$$

Here C is a constant of integration, and, if the initial blood flow rate when t=0 is termed $Q_0$, then the relationship $C=1/Q_0$ holds.

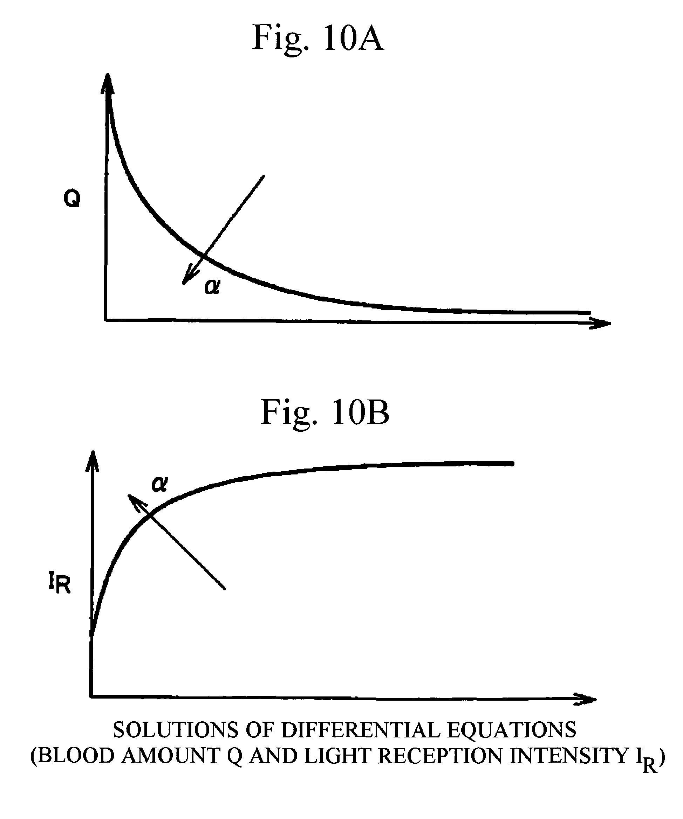

Q and the light reception intensity IR observed at this time are shown in Fig. 10. While $\alpha$ is a parameter that determines the form of the function, as will be understood from Equation (10), this $\alpha$ is proportional to the reciprocal of the viscosity $\mu$, and from this fact it will be understood that it is possible to evaluate the blood viscosity by estimating the value of the parameter $\alpha$ from the measured data for the amount of received light.

Next, the physical meaning of the parameter $\alpha$ will be investigated.

The parameter $\alpha$ in the equation of motion (9) that gives the change of the amount of blood when the pressure member 10 is pressed against the test site 100 is given by Equation (10):

$$\alpha = \frac{P_a}{8\pi n^2 l_m^2 \mu} \quad \cdot \cdot \cdot (1\,0)$$

As will be clear from the differential equation (9) as well, this parameter gives the "rate of decrease of the blood amount Q (due to the application of pressure)", but let us try to enumerate the causes that determine this parameter.

    1. The blood viscosity $\mu$:

        When the viscosity is high the blood loses its fluidity, and $\alpha$ becomes small. On the other hand, the smaller is the viscosity (in other words, the higher is the blood fluidity), the larger $\alpha$ becomes.

    2. n and $l_m$:

        Now, if the total capacity of the capillaries (i.e. the total blood amount) before application of pressure is termed $Q_0$, then, since the relationship in the following Equation (18)

$$Q_0 = \pi R_l^2 n l_m \quad \cdot \cdot \cdot (1\,8)$$

        holds (here $R_1$ is the radius of the blood vessels before application of pressure), accordingly the following Equation (19) holds:

$$n l_m = \frac{Q_0}{\pi R_l^2} \quad \cdot \cdot \cdot (1\,9)$$

        n and $l_m$ are parameters that correspond to the total elongation of the blood vessels, and they do not depend upon the total amount of blood in the capillary system [as will also be understood from the fact that, in Equation (19), they are divided by the internal diameter term]. In other words, they are parameters that depend upon the area to which the pressure is applied.

3. The applied pressure $P_a$:

When the applied pressure $P_a$ becomes great, $\alpha$ becomes great. In order to eliminate the influence of $P_a$, it will be effective always to apply a constant pressure, or to use a value $\alpha_N$ normalized by $P_a$ instead of $\alpha$, as in the following Equation (20):

$$\alpha_N = \alpha / P_a \quad \cdots (2\,0)$$

Next, let us consider the method by which $\alpha$ is estimated from the light reception intensity $I_R$ that is observed when pressure is applied.

When the total blood amount Q is given by Equation (17), and its waveform is given by Fig. 10, then the light reception intensity $I_R$ is given by the following Equation (21):

$$I_r = I_{R,m} - \frac{1}{\alpha t + \dfrac{1}{I_{R,m}}} \quad \cdots (2\,1)$$

Here, $I_{R,m}$ is the maximum value of the light reception intensity.

When Equation (21) is differentiated by time, the following Equation (22) is obtained:

$$\frac{\partial I_R}{\partial t} = \frac{\alpha}{\left(\alpha t + \dfrac{1}{I_{R,m}}\right)^2} \quad \cdots (2\,2)$$

The value of the differential coefficient at the time point t=0 is given by the following

Equation (23):

$$\frac{\partial I_R}{\partial t}\bigg|_{t=0} = \alpha I_{R,m}{}^2 = K_0 \quad \cdots (2\,3)$$

And the value at the time point t=1 (seconds) is given by the following Equation (24):

$$\frac{\partial I_R}{\partial t}\bigg|_{t=\sec} = \frac{\alpha}{\left(\alpha + \dfrac{1}{I_{R,m}}\right)^2} = \frac{\alpha I_{R,m}{}^2}{(\alpha I_{R,m} + 1)^2} = K_1 \quad \cdots (2\,4)$$

Furthermore, according to Equation (22), if $I_{R,m}$ is standardized as 1, the following Equation (25) is obtained:

$$\alpha = \frac{\partial I_R}{\partial t}\bigg|_{t=0} \quad \cdots (2\,5)$$

In other words, when standardized by the maximum value of the light reception intensity, $\alpha$ is equal to the initial slope.

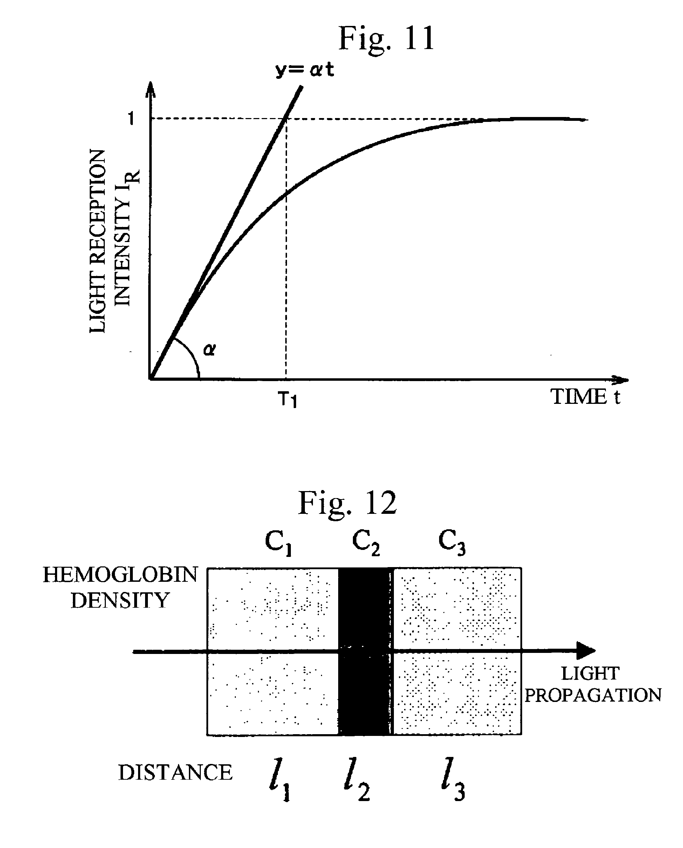

As shown in Equation (25), since $\alpha$ is a coefficient that is minute at the time point t=0 if the maximum value of the light reception intensity is standardized at 1, this may be shown as in Fig. 11.

Here, the time point $T_1$ at which the straight line y=$\alpha$t meets y=1 is given by the following Equation (26):

$$T_1 = \frac{1}{\alpha} \qquad \cdots (2\,6)$$

and this $T_1$ is also a parameter that is correlated with blood viscosity. In other words, $T_1$ (which is equal to the reciprocal of $\alpha$) is the time interval over which all the blood would flow out, if it is supposed that the initial outflow speed of blood (=$\alpha$) were to continue at a constant value; and, when the definition equation for $\alpha$ is used for this $T_1$, the following Equation (27) is obtained:

$$T_1 = \frac{8\pi n^2 l_m^2}{P_a}\mu \qquad (\text{sec}) \qquad \cdots (2\,7)$$

so that $T_1$ is a parameter that is proportional to the blood viscosity $\mu$.

Furthermore, when we obtain the value of the differential coefficient at the time point t=n (in seconds), the following Equation (28) results:

$$\frac{\partial I_R}{\partial t}\bigg|_{t=n\,\text{sec}} = \frac{\alpha I_{R,m}^2}{(nI_{R,m}\alpha + 1)^2} \qquad \cdots (2\,8)$$

And, when Equation (23) is considered, Equation (29) is obtained:

$$\alpha = \frac{\left(\sqrt{K_0} - \sqrt{K_n}\right)^2}{n^2 K_0 K_n} \qquad \cdots (2\,9)$$

As may be understood from this Equation (29), even if the maximum value $I_{R,m}$ of the light reception intensity cannot be measured, if the initial value $K_0$ of the differential coefficient and its value $K_n$ at any time point t=n are known, then it is possible to obtain $\alpha$ from those values, and it is possible to evaluate the blood viscosity from this value of $\alpha$.

Next, a blood fluidity evaluation method according to another embodiment of the present invention will be explained.

The blood fluidity evaluation method according to this embodiment is distinguished by the fact that the hemoglobin density within the tissue (i.e. a value from 0 to 1 that is the proportional amount of hemoglobin when the mass of the entire tissue is taken as 1) at the site at which pressure has been applied is calculated from the light intensity data that is received from the light reception element 12, and the fluidity of the blood is evaluated on the basis of the change over time of this hemoglobin density.

As one example of the method of evaluation, the viscosity of the blood, which is one element in the fluidity of the blood, is evaluated on the basis of the time period T(=$t_2$-$t_1$) that is required for the hemoglobin density to decrease from a stage at which it has decreased to a first predetermined value (for example 2% or 1 %) that is sufficiently smaller than its value before the pressure was applied, to a second predetermined value that is yet smaller (for example half of the first predetermined value, i.e. 1% or 0.5%).

Furthermore, as the next stage, a blood viscosity evaluation parameter is calculated from the light intensity data received from the light reception element 12, and the relationship between this blood viscosity evaluation parameter and the above hemoglobin density is displayed upon a graph, with the blood viscosity evaluation parameter shown along the vertical axis and the hemoglobin density shown along the horizontal axis.

Generally, it is considered that the viscosity of blood increases due to reduction of the deformability of the red blood cells, due to the agglomeration of the red blood cells, due to increase of the hematocrit value (i.e. increase of the volume % of the red blood cells), changes of the viscoelastic characteristics of the white blood cells and of the blood plasma, and so on. Furthermore when the shear rate of blood becomes great its viscosity greatly decreases, which is a so-called rheological or thixotropic phenomenon. It is considered that, if it is possible to measure the hemoglobin density within the capillaries of blood having this type of characteristic quantitatively, then it will be possible to estimate the viscosity of the blood from this datum. A large number of capillaries are present in the surface skin of a human being, and their presence governs the direct circulation of blood to the skin cells. When a pressure that is greater than or equal to the blood pressure in these capillaries at the surface of the skin is applied, then the blood flows out from the capillaries, and the amount of blood in the capillaries underneath the skin to which the pressure has been applied gradually diminishes. This phenomenon of blood flowing out from the capillaries occurs due to the diameters of these blood vessels becoming smaller because of the application of pressure, and due to the blood being pressed out from them.

However, since capillaries are extremely small in diameter, the amount of blood that can flow out of them is limited, and blood flows out slowly to the other parts of the circulatory system to which pressure is not being applied. Moreover due to the fact that, as previously described, the diameter of these blood vessels has become minute, accordingly changes of viscosity occur because of agglomeration of the red blood cells and reduction of their deformability, and, thanks to this influence, the amount of outflow of blood from the capillaries progressively decreases under pressure along with the passage of time. In other words, to put the matter conversely, it becomes possible to measure the viscosity of the blood by observing the process of outflow of blood in the capillaries under the application of pressure.

When the optical measurement from outside the skin of the subject of the outflow of blood is considered, visible light is greatly absorbed by the hemoglobin in the living body and by the other structural substances that make up a living body, and hardly can pass through at all. However, when the blood in the capillaries is caused to flow out of them by application of pressure to the skin, then the amount of blood (i.e. of hemoglobin) is reduced along therewith, and the amount of absorption of light in the body of the subject is reduced as compared to its value before pressure application. In other words, blood in the capillaries flows out due to the application of pressure, and the light passes through somewhat better as the amount of blood (i.e. of hemoglobin) becomes less. When the wavelength of the light is considered, light of short wavelength is hardly transmitted through a living body at all. In other words, by observing light of short wavelength irradiated against a living body, it may be considered that the light that is being observed is light that has been propagated through the capillaries directly under the skin. By taking advantage of this theory, it becomes possible to observe the state in which the blood within the capillaries flows out due to the application of pressure, and from this it becomes possible to measure the hemoglobin density.

Next, we attempt to investigate quantification of the hemoglobin density.

Basically the incident light arrives at the detector after having been multiply scattered within the tissue of the body of the subject, but in order to simplify this here, it may be considered that the light arrives at the detector along some optical path. Here, the length of this equivalent optical path is supposed to be I (lowercase letter "L"). Moreover, it may be considered that this optical path remains always fixed, irrespective of the presence or absence o blood in the capillaries. According to Lambert-Beer's law, attenuation due to the hemoglobin in blood is given by Equation (30) below:

$$A_B = \exp(-\varepsilon c \ell) \qquad (30)$$

Here:

$\varepsilon$ is the extinction coefficient of hemoglobin at the wavelength of the light concerned (units $mm^{-1}$);
and c is the density of hemoglobin in the tissues (a dimensionless quantity whose value is between 0 and 1).

We employ the term $A_T$ for the attenuation of light by living tissue. Apart from the attenuation by tissue, this attenuation also includes attenuation by components of the blood that remain in the tissue even after pressure has been applied for an indefinitely long period of time. Moreover, we use the term K for the efficiency of detection by the detector.

At this time, in relation to the output $I_r$ of the detector: according to the presence or absence of application of pressure to the blood in the capillary system, (a) the detector output $I_{rB}$ directly before the application of pressure (i.e. in the state in which blood is present) is given by the following Equation (31):

$$I_{rB} = K I_0 A_T \exp(-\varepsilon c \ell) + I_D \qquad (31)$$

Here, $I_D$ is the light directly propagated between the light source and the detector (including also external light).

On the other hand, (b) the detector output $I_{rT}$ after a sufficiently long period of time has elapsed from the application of pressure (or its value after a sufficiently long time period has elapsed as estimated by an appropriate bias estimation method) can be expressed as in the following Equation (32):

$$I_{rT} = K I_0 A_T + I_D \qquad (32)$$

If we suppose that it has been possible to measure the level of direct light $I_D$, then the values obtained by subtracting this from Equations (31) and (32) may be expressed by the following Equations (33) and (34):

$$I'_{rB} = I_{rB} - I_D = K I_0 A_T \exp(-\varepsilon c \ell) \qquad (33)$$

$$I'_{rT} = I_{rT} - I_D = K I_0 A_T \qquad (34)$$

When the logarithms of both sides of Equations (33) and (34) are taken, the following Equations (35) and (36) are obtained:

$$\ln(I'_{rB}) = \ln(K I_0 A_T) - \varepsilon c \ell \qquad (35)$$

$$\ln(I'_{rT}) = \ln(K I_0 A_T) \qquad (36)$$

Accordingly, it is possible to derive the following Equation (37):

$$\varepsilon c \ell = \ln(I'_{rT}) - \ln(I'_{rB}) = \ln\left(\frac{I'_{rT}}{I'_{rB}}\right) \qquad (37)$$

Due to this, supposing that the equivalent optical path length $l$ and the extinction coefficient $\varepsilon$ of hemoglobin are already known, the hemoglobin density c may be obtained from Equation (38) below:

$$c = \frac{\ln\left(\dfrac{I'_{rT}}{I'_{rB}}\right)}{\varepsilon \ell} \qquad (38)$$

For example, while the amount of hemoglobin in the tissues decreases directly after the application of pressure along with decrease of the amount of blood, if the density of hemoglobin at some time point is termed $c_i$ and the intensity of the light received by the light reception element at that time point is termed $I_{rBi}$, then this hemoglobin density $c_i$ at this time point may be estimated from the following Equation (39). However, here it is considered that there is no influence due to the directly propagated light $I_D$. The following Equation (39)

$$c_i = \frac{1}{\varepsilon l} \ln\left(\frac{I_{rT}}{I_{rBi}}\right) \qquad (39)$$

is a general purpose equation for measuring the hemoglobin density, from which the hemoglobin density $c_i$ along the light propagation path can be estimated.

Generally, it is considered that layers of differing hemoglobin density, such as epidermis, dermis, subcutaneous tissue, and so on, are superimposed over one another along the path upon which the light is obscured. Fig. 12 is a schematic figure showing the propagation of light through a plurality of layers of differing hemoglobin densities. In this case, the light reception intensity when layers of thickness $l_i$ and having hemoglobin density $c_i$ are superimposed in sequence along the light propagation path may be expressed by the following Equation (40):

$$I_{rB} = KI_0 A_r \exp\left(-\sum_i \varepsilon_i c_i l_i\right) \qquad (40)$$

Here, $\varepsilon_i$ is the extinction coefficient in the i-th layer, $c_i$ is the hemoglobin density in the i-th layer, and $l_i$ is the thickness of the i-th layer.

In this case as well, the intensity of the light that has passed through the previous layer after a sufficient time period has elapsed from the application of pressure (actually, the final value of the light intensity after estimation, i.e. the bias value) may be expressed by Equation (41) below:

$$\sum_i \varepsilon_i c_i l_i = \ln\left(\frac{I_{rT}}{I_{rB}}\right) \qquad (41)$$

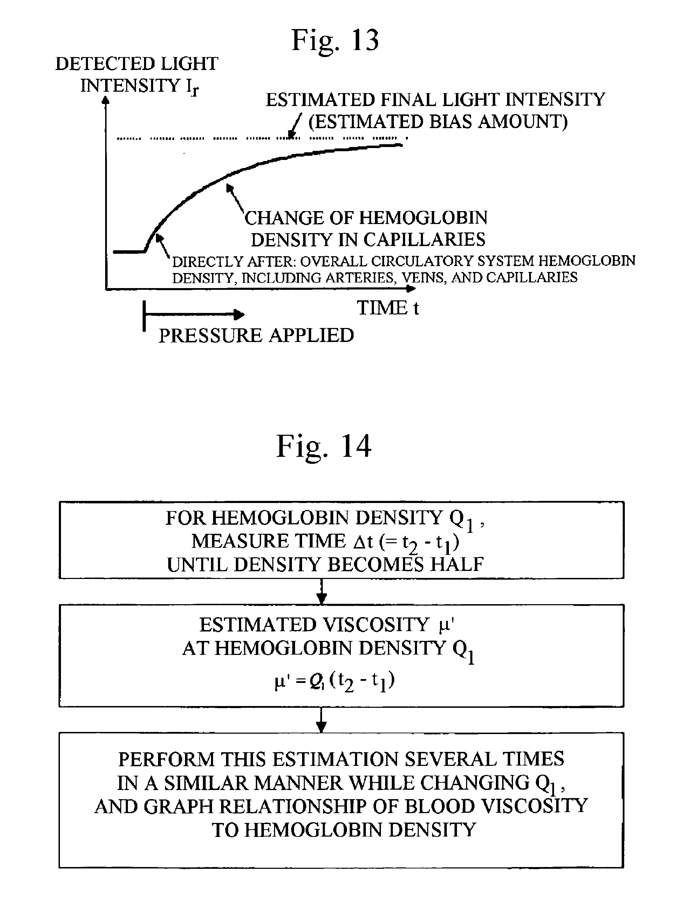

so that it is possible to estimate the weighted average value of the hemoglobin amount. However skin has a layered construction including epidermis, dermis, and subcutaneous tissue, and since, in the circulatory system, the blood in the small arteries and veins whose blood vessel resistance is small flows out rapidly directly after the application of pressure, accordingly, after a certain time period has elapsed, the hemoglobin amount that can be estimated with Equation (41) may be considered as being only due to the capillaries. Fig. 13 summarizes this situation.

Next, a method will be described of estimating the viscosity of the blood from the time interval required for the hemoglobin density to decrease by a predetermined amount. While, in the embodiment described above, as a method for estimating the viscosity of the blood by utilizing the light reception intensity, a method [the method obtained from Equation (9)] was mentioned of obtaining blood viscosity from the differential with respect to time of the light reception intensity Q (= the blood amount), in this embodiment, the viscosity of the blood is obtained from the time required for the hemoglobin density to decrease.

The change over time of the blood amount Q (here the hematocrit value is considered as constant, so it is replaced by the hemoglobin density) may be expressed by the following Equation (42):

$$Q = \frac{1}{\alpha t + \frac{1}{Q_0}} = \frac{Q_0}{\alpha Q_0 t + 1} \qquad (42)$$

Here, $Q_0$ is the amount of blood before pressure application (considered as the hemoglobin density),

$$\alpha = \frac{P_a}{8\pi n^2 \ell_m^2 \mu} = K\frac{1}{\mu} \quad,$$

and

$\mu$ is the blood viscosity.

Now, if the hemoglobin density at time t1 is $Q_1$ and the hemoglobin density at time t2 is $Q_2$, then from Equation (42), the following Equations (43) can be derived:

$$Q_1 = \frac{Q_0}{\alpha Q_0 t_1 + 1}$$

$$Q_2 = \frac{Q_0}{\alpha Q_0 t_2 + 1} \qquad (43)$$

From these equations, via Equations (44), Equation (45) can be derived:

$$t_1 = \frac{Q_0 - Q_1}{\alpha Q_0 Q_1}$$

$$t_2 = \frac{Q_0 - Q_2}{\alpha Q_0 Q_2} \qquad (44)$$

$$t_2 - t_1 = \frac{1}{\alpha}\left(\frac{Q_0 - Q_2}{Q_0 Q_2} - \frac{Q_0 - Q_1}{Q_0 Q_1}\right) = \frac{1}{\alpha}\left(\frac{Q_1 - Q_2}{Q_1 Q_2}\right) \qquad (45)$$

In other words the parameter $\alpha$, which is related to the reciprocal of the viscosity $\mu$, can be expressed by Equation (46) below:

$$\alpha = \frac{Q_1 - Q_2}{Q_1 Q_2} \cdot \frac{1}{t_2 - t_1} \qquad (46)$$

Or, the parameter $\mu'$ which is proportional to the viscosity $\mu$ can be expressed by Equation (47) below:

$$\mu' = \frac{1}{\alpha} = \frac{Q_1 Q_2}{Q_1 - Q_2}(t_2 - t_1) \qquad (47)$$

Here, to consider the viscous correlation parameter p': if the hemoglobin density Q is in % and the time t is in seconds, then its units are %/sec. This gives the blood viscosity between the hemoglobin densities $Q_1$ and $Q_2$, and the larger its value is, the larger is the viscosity. For example, the case $Q_2 = 0.5 Q_1$ may be considered. In this case, Equation (47) becomes Equation (48) below:

$$\mu' = Q_1(t_2 - t_1) \qquad (48)$$

In other words, if the hemoglobin density $Q_1$ is taken as being 2%, then it is possible to estimate the blood viscosity when the hemoglobin density is 2% using Equation (48), after having measured the time interval $(t_2-t_1)$ that it takes for this density to drop to half, i.e. to 1%.
In the same manner, if the hemoglobin density $Q_1$ is taken as being 1%, then it is possible to estimate the blood viscosity when the hemoglobin density is 1% using Equation (48), after having measured the time interval $(t_2-t_1)$ that it takes for this density to drop to half, i.e. to 0.5%. The longer is the time needed for the hemoglobin density to decrease to half, the higher is the viscosity.
A flow chart for the estimation of the blood viscosity correlation parameter $\mu'$ is schematically shown in Fig. 14.
First, in a first step, for the hemoglobin density of $Q_1$, the time interval $(t_2-t_1)$ for the density to drop to half is measured. Next, in a second step, the viscosity $\mu'$ at the hemoglobin density of $Q_1$ is estimated according to Equation (48). And then, in a third step, the above estimation is performed several more times while varying the hemoglobin density $Q_1$, and the relationship "hemoglobin density" versus "blood viscosity parameter" is displayed upon a graph.
Now, the time interval $(t_2-t_1)$ for the hemoglobin density to drop from $Q_1$ by half so as to become $0.5 Q_1$ is given by Equation (49):

$$(t_2 - t_1) = \frac{1}{\alpha Q_1} \qquad (49)$$

Next the meaning of the time interval $(t_2-t_1)$ will be investigated. If now at the time instant t=0 the hemoglobin density is $Q_1$, then its change with time can be expressed by Equation (50):

$$Q = \frac{Q_1}{\alpha Q_1 t + 1} \qquad (50)$$

By differentiating this equation with respect to t, the following Equation (51) can be derived:

$$\frac{dQ}{dt} = -\frac{\alpha Q_1^2}{(\alpha Q_1 t + 1)^2} \qquad (51)$$

At t=0 (in other words, when the amount of hemoglobin is Q1), this differential value may be expressed by Equation (52):

$$\frac{dQ}{dt}\bigg|_{t=0} = -\alpha Q_1^2 \qquad (52)$$

And, when the tangent line for the hemoglobin amount Q having the inclination of Equation (52) and having the value $Q_1$ at t=0 is considered, then Equation (53) can be derived:

$$Q = -\alpha Q_1^2 t + Q_1 \qquad (53)$$

The time point T at which this tangent line intersects Q=0 may be obtained from Equation (54):

$$T = \frac{1}{\alpha Q_1} = (t_2 - t_1) \qquad (54)$$

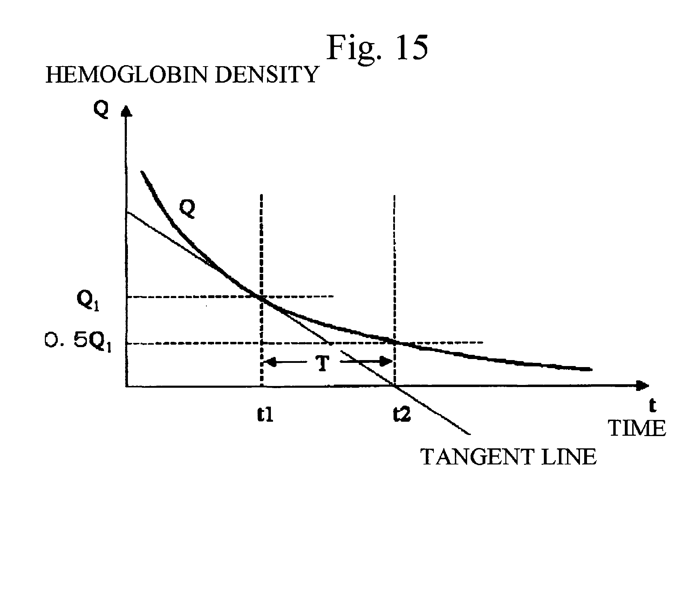

Fig. 15 schematically shows the relationship described above.

From this relationship, the time interval $(t_2-t_1)$ needed for the hemoglobin density to become half is found to have the following three meanings:

(1) $(t_2-t_1)$ is the time period required for the hemoglobin density Q to become half its original value (the fundamental meaning);

(2) Or, its meaning may be attached to $\alpha$ or to the viscosity parameter $\mu$' (refer to Equations (55) and (56) below);

$$\alpha = \frac{1}{Q_1(t_2 - t_1)} \qquad (55)$$

$$\mu' = \frac{1}{\alpha} = Q_1(t_2 - t_1) \qquad (56)$$

(3) Or, it is the time interval T (=$t_2$-$t_1$) required for the tangent line to the hemoglobin density $Q_1$ to reach Q=0.

In other words, T is the time period that would be required for all of the blood to flow out, under the supposition that the blood outflow continues unabated (i.e. that the outflow speed when $Q=Q_1$ is always maintained).

As an example the flow of processing proceeds as follows.

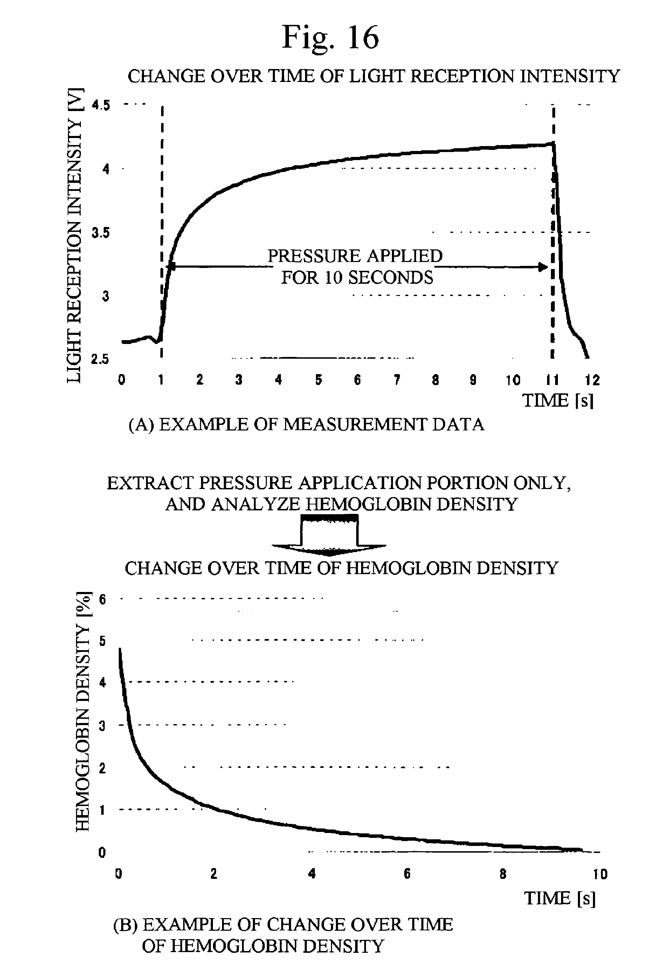

(1) The light reception intensity measurements are displayed upon a graph, as in Fig. 16.

(2) The light reception intensity after an infinite time has elapsed is estimated (actually, some point of the measured values is considered to be the fictitious convergence value, and the difference between the convergence value at infinite time of the blood amount in the model equation and this fictitious convergence value, in other words the bias value, is obtained).

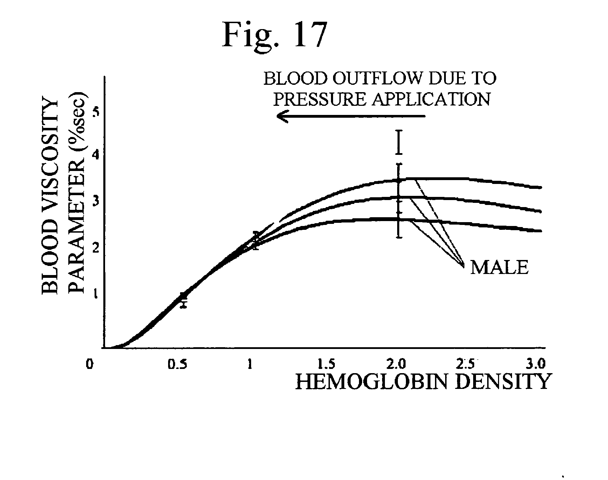

(3) The hemoglobin density is calculated (the hemoglobin density is calculated according to Lambert-Beer's Law from the light reception intensity directly after application of pressure and the light reception intensity when a sufficiently large time period has elapsed after application of pressure). $C_1$, $C_2$, and $C_3$ in Fig. 17 correspond to hemoglobin density.

(4) The blood viscosity parameter is calculated (the blood viscosity parameter $\mu'$ ($\mu'=1/\alpha$) is derived from the time taken until the hemoglobin density reaches 1/2 of its original value).

Attention is directed to the portion where the waveform converges, and, from the model equation for blood outflow from the capillaries, a portion where influence from the blood pressure or from AVR (artery-vein shunting) cannot easily be experienced is selected, and the blood viscosity is evaluated. Since the blood resistance of capillaries is high, the blood flows out slowly even when pressure is applied, and it takes a long time before these blood vessels become closed.

When the hemoglobin density is great, the graph of density change exhibits great deviations due to conditions of various types, but the graph converges when the hemoglobin density is 2% or less. Thus, the change at the stage that the hemoglobin density is 2% or less is investigated. This is because, in this case, it is possible to eliminate the variations directly after the application of pressure.

The following advantageous effects are obtained by evaluating the viscosity of the blood of the subject while standardizing the hemoglobin density in this manner. That is, for example, while the density of hemoglobin in the tissues changes along with elapsed time from the application of pressure (i.e. changes along with outflow of the blood), if the viscosity of the blood is evaluated on the basis of change of the hemoglobin density at a stage at which that density is great, then it is possible to become aware of the viscosity characteristic of the entire mass of blood while it is flowing as a thick layer flow that consists, in particular, of red blood cells and blood plasma and so on (it is considered that this corresponds to the range to which the blood is flowing thickly, since it is also correlated with the so-called coefficient of kinematic viscosity). Furthermore, if the viscosity of the blood is evaluated on the basis of change of the density of hemoglobin within the tissues when that density has become sufficiently small, then it is possible to become aware of the viscosity characteristic of the blood at a stage at which changes are reflected therein that can deform the red blood cells, due to the diameters of the blood vessels such as the capillaries having become sufficiently small.

Moreover, since the density of hemoglobin within the tissues may be termed a "parameter that determines the quality of the blood circulation", it is possible to anticipate that measuring (or estimating) this hemoglobin density will be useful for evaluating the likelihood of the danger of bedsores occurring due to impeded circulation in the capillary system, and for preventing frostbite (including light frostbite like chilblains), for evaluation of skin regrowth after external damage, and so on.

Yet further, by evaluating the viscosity of the blood on the basis of the time period needed for the hemoglobin density to drop to half its original value, it is possible to perform blood viscosity evaluation in a short period of time during a stage in which a sufficient amount of blood is flowing out from the capillary system, in other words during a stage in which no influence from artery-vein shunting is experienced.

Furthermore, by displaying the relationship between the blood viscosity evaluation parameter and the density of hemoglobin upon a single graph as shown in Fig. 17, not only is possible to display only the necessary level without experiencing any influence from artery-vein shunting, but also it is possible to evaluate the viscosity, irrespective of differences in the initial blood amount or in the time that has elapsed from when pressure was applied. Moreover, according to this graph, it also becomes possible clearly to understand at a glance the blood rheology [i.e. the way in which the blood viscosity changes according to the diameter of the blood vessels (which here corresponds to the hemoglobin amount)].

According to the present invention, it is possible to evaluate the fluidity of the blood of a subject from the exterior of his body in a simple manner, so that it is possible to respond to demands for maintenance of human health.

While the invention has been described with respect to specific examples including presently preferred modes of carrying out the invention, those skilled in the art will appreciate that there are numerous variations of the above described devices and techniques that fall within the scope of the invention as set forth in the appended claims.

**Claims**

1. A blood fluidity evaluation method in which a pressure member is pressed against a test site on a test subject, blood at the test site is squeezed and flows out to the periphery of the test site, change over time of the amount of blood at the test site is measured at this time using optical scattering, and blood fluidity at the test site is evaluated from this measurement data, **characterized in that** the pulse of the test subject is measured at said test site, and driving of said pressure member is controlled on the basis of said pulse measurement data.

2. A blood fluidity evaluation method in which a pressure member is pressed against a test site on a test subject, blood at the test site is squeezed and flows out to the periphery of the test site, change over time of the amount of blood at the test site is measured at this time using optical scattering, and blood fluidity at the test site is evaluated from this measurement data, **characterized in that** the pulse of the test subject is measured at a pulse measurement site that is different from the test site, and driving of said pressure member is controlled on the basis of said pulse measurement data.

3. The blood fluidity evaluation method according to Claim 1 or 2, wherein, before the measurement described above, the following positioning process is performed: while shifting the relative position of the pressure member with respect to the test site, the pressure member is pressed against the test site with a weak force sufficient to squeeze blood at the test site to flow out to the periphery of the test site, pulse measurement data is acquired due to the absorption of light; and a relative position, at which the intensity of the pulse measurement data attains a local maximum, is determined as being an optimum measurement position.

4. The blood fluidity evaluation method according to Claim 1, 2 or 3, wherein the time at which the driving of said pressure is started is determined on the basis of said pulse measurement data.

5. A blood fluidity evaluation device (1) comprising:

   a pressure member (10) that can be pressed against a test site (100) upon a test subject;
   a drive control device that controls and drives said pressure member;
   a light emission element (11) that is installed upon said pressure member and that irradiates light onto the test site;
   a light reception elemen (12) that is installed upon said pressure member and that detects light scattered within the test site;
   a calculation means (25) that calculates the amount of blood and the blood fluidity at the test site from the measurement data from said light reception element; and
   a display means (27) that displays at least one of the blood amount and the blood fluidity,
   the blood fluidity evaluation device **characterized by** further comprising a pulse measurement means (31) that measures the pulse of the test subject at a pulse measurement site (110) that is different from the test site; and
   wherein said drive control device is adapted to control driving of said pressure member on the basis of measurement data from said pulse measurement means.

**Patentansprüche**

1. Ein Verfahren zur Auswertung der Fliessfähigkeit des Bluts, bei dem ein Druckelement gegen eine Teststelle auf einem Testgegenstand gedrückt wird, Blut an der Teststelle gedrückt wird und zum Umfeld der Teststelle ausfliesst, in dieser Zeit mit Hilfe der optischen Streuung die Änderung über die Zeit der Menge des Bluts an der Teststelle gemessen wird, und die Fliessfähigkeit des Bluts an der Teststelle aus dieser Messdaten ausgewertet werden, **dadurch gekennzeichnet, dass** der Puls des Testgegenstands an der Teststelle gemessen wird, und der Antrieb des Druckelements auf Basis von Pulsmessungsdaten gesteuert wird.

2. Ein Verfahren zur Auswertung der Fliessfähigkeit des Bluts, bei dem ein Druckelement gegen eine Teststelle auf einem Testgegenstand gedrückt wird, Blut an der Teststelle gedrückt wird und zum Umfeld der Teststelle ausfliesst, in dieser Zeit mit Hilfe der optischen Streuung die Änderung über die Zeit der Menge des Bluts an der Teststelle gemessen wird, und die Fliessfähigkeit des Bluts an der Teststelle aus dieser Messdaten ausgewertet werden, **dadurch gekennzeichnet, dass** der Puls des Testgegenstands an der Pulsmessungsstelle gemessen wird, und der Antrieb des Drukkelements auf Basis von Pulsmessungsdaten gesteuert wird.

3. Das Verfahren zur Auswertung der Fliessfähigkeit des Bluts gemäss dem Anspruch 1 oder 2, wobei vor der oben beschriebenen Messung, folgender Positionierungsvorgang durchgeführt wird: während die relative Position des Druckelements in Bezug auf die Teststelle verschoben wird, wird das Druckelement gegen die Teststelle mit einer schwachen Kraft gedrückt, die ausreicht, um Blut an der Teststelle gedrückt wird und zum Umfeld der Teststelle ausfliesst, Pulsmessungsdaten aufgrund der Absorption von Licht erfasst werden; und eine relative Position, bei der die Intensität der Pulsmessungsdaten ein lokales Maximum erreicht, wird als eine optimale Messposition ermittelt.

4. Das Verfahren zur Auswertung der Fliessfähigkeit des Bluts gemäss dem Anspruch 1, 2 oder 3, wobei die Zeit, bei der die Ansteuerung des Drucks gestartet wird, wird auf der Grundlage der Pulsmessungsdaten bestimmt.

**5.** Eine Einrichtung zur Auswertung der Fliessfähigkeit des Bluts, umfassend:

ein Druckelement, das gegen eine Teststelle auf einem Testgegenstand gedrückt werden kann;
eine Antriebssteuerungseinrichtung, die das Druckelement steuert und treibt;
ein Licht emittierendes Element, das auf dem Druckelement installiert ist und auf die Teststelle Licht strahlt;
ein Lichtempfangselement, das auf dem Druckelement installiert ist und innerhalb der Teststelle gestreutes Licht erkennt;
ein Berechnungsmittel, dass die Menge von Blut und die Fliessfähigkeit des Bluts an der Teststelle aus den Messdaten des Lichtempfangselements berechnet; und
eine Anzeigeeinrichtung, die mindestens eine der Blutmenge und Fliessfähigkeit des Bluts anzeigt,
die Einrichtung zur Auswertung der Fliessfähigkeit des Bluts **dadurch gekennzeichnet** ferner umfassend ein Pulsmessungsmittel, das den Puls des Testgegenstands bei einer Pulsmessungsstelle misst, die anders als die Teststelle ist; und wobei die Antriebssteuerungseinrichtung so ausgelegt ist, dass der Antrieb des Druckelements auf der Grundlage von Messdaten des Pulsmessungsmittels gesteuert wird.

**Revendications**

**1.** Procédé d'évaluation de fluidité du sang, dans lequel un élément de pression est pressé contre un site de test sur un sujet d'essai, le sang au niveau du site de test est pressé et s'écoule vers la périphérie de la zone d'essai, changer avec le temps de la quantité de sang à l'essai site est mesurée à ce moment en utilisant la diffusion optique, et la fluidité du sang sur le site de test est évaluée à partir de ces données de mesure, **caractérisé en ce que** l'impulsion du sujet d'essai est mesurée au niveau dudit site d'essai, et d'entraînement dudit élément de pression est commandée sur la base de ladite impulsion de données de mesure.

**2.** Procédé d'évaluation de fluidité du sang, dans lequel un élément de pression est pressé contre un site de test sur un sujet d'essai, le sang au niveau du site de test est pressé et s'écoule vers la périphérie de la zone d'essai, changer avec le temps de la quantité de sang à l'essai site est mesurée à ce moment en utilisant la diffusion optique, et la fluidité du sang sur le site de test est évaluée à partir de ces données de mesure, **caractérisé en ce que** l'impulsion du sujet d'essai est mesurée à un site de mesure d'impulsions qui est différent du site de test, et d'entraînement dudit élément de pression est commandée sur la base de ladite impulsion de mesure des données.

**3.** Procédé d'évaluation du sang fluidité selon la revendication 1 ou la revendication 2, dans lequel, avant la mesure décrite ci-dessus, le processus de positionnement suivant est effectué: tout en déplaçant la position relative de l'élément de pression par rapport au site d'essai, l'élément de pression est pressé contre le site d'essai avec une force faible suffisante pour presser le sang au niveau du site de test de s'écouler hors de la périphérie de la zone d'essai, d'impulsions de mesure de données est acquise en raison de l'absorption de la lumière, et une position relative, à laquelle l'intensité des données de mesure d'impulsions atteint un maximum local, est déterminée comme étant une position de mesure optimale.

**4.** Procédé d'évaluation du sang fluidité selon la revendication 1, 2 ou 3, dans lequel l'heure à laquelle l'entraînement de ladite pression est démarré est déterminée sur la base de ladite impulsion de données de mesure.

**5.** Un dispositif d'évaluation du sang fluidité, comprenant:

un élément de pression pouvant être pressé contre un site d'essai sur un sujet d'essai;
un dispositif de commande d'entraînement qui commande et entraîne ledit élément de pression;
un élément d'émission de lumière qui est installé sur ledit élément de pression et qui irradie de la lumière sur le site d'essai;
un élément de réception de lumière qui est installé sur ledit élément de pression et qui détecte la lumière diffusée dans le site d'essai;
un moyen de calcul qui calcule la quantité de sang et la fluidité du sang au niveau du site de test à partir des données de mesure provenant dudit élément de réception de lumière, et
un moyen d'affichage qui affiche au moins un de la quantité de sang et la fluidité du sang,
le dispositif d'évaluation du sang fluidité caractérisé en outre comprenant un moyen de mesure du pouls qui mesure l'impulsion du sujet d'essai à un site de mesure d'impulsions qui est différent du site de test, et dans lequel ledit dispositif de commande d'entraînement est adapté pour commander l'entraînement dudit élément de pression sur la base de données de mesure de ladite impulsion de moyens de mesure.

# Fig. 1

100

12

10

1

11

21

PRESSURE DEVICE

LIGHT EMISSION
ELEMENT DRIVE
CIRCUIT

RECEIVED OPTICAL
SIGNAL DETECTION
CIRCUIT

24

PRESSURE DEVICE
DRIVE CIRCUIT

23

22

CPU

25

OPERATION SWITCH

DISPLAY DEVICE

26

27

# Fig. 2

# Fig. 3

## CHANGE OF RECEIVED AMOUNT LIGHT
## WHEN BLOOD VISCOSITY IS LOW

# Fig. 4

CHANGE OF RECEIVED AMOUNT LIGHT
WHEN BLOOD VISCOSITY IS HIGH

PRESSURE RELEASED

# Fig. 5

ARTERIAL MODEL

# Fig. 6

INFLUENCE OF GRAVITY
DUE TO ATTITUDE

Ro

C

PRESSURE
UPON TISSUE

ARTERIAL MODEL

# Fig. 7A

CAPILLARY SYSTEM
MODEL 1

Ro

C

# Fig. 7B

CAPILLARY SYSTEM
MODEL 2

Ro

C

# Fig. 8

CAPILLARY SYSTEM
WHEN PRESSURE APPLIED

# Fig. 9

KINETIC STATE OF BLOOD VESSELS UPON
PRESSURE APPLICATION FROM EXTERIOR

# Fig. 10A

# Fig. 10B

SOLUTIONS OF DIFFERENTIAL EQUATIONS
(BLOOD AMOUNT Q AND LIGHT RECEPTION INTENSITY $I_R$)

# Fig. 11

$y = \alpha t$

LIGHT RECEPTION
INTENSITY $I_R$

1

$\alpha$

$T_1$

TIME t

# Fig. 12

$C_1$    $C_2$    $C_3$

HEMOGLOBIN
DENSITY

LIGHT
PROPAGATION

DISTANCE    $l_1$    $l_2$    $l_3$

## Fig. 13

DETECTED LIGHT
INTENSITY $I_r$

ESTIMATED FINAL LIGHT INTENSITY
(ESTIMATED BIAS AMOUNT)

CHANGE OF HEMOGLOBIN
DENSITY IN CAPILLARIES

DIRECTLY AFTER: OVERALL CIRCULATORY SYSTEM HEMOGLOBIN
DENSITY, INCLUDING ARTERIES, VEINS, AND CAPILLARIES

TIME t

PRESSURE APPLIED

## Fig. 14

FOR HEMOGLOBIN DENSITY $Q_1$,
MEASURE TIME $\Delta t (= t_2 - t_1)$
UNTIL DENSITY BECOMES HALF

ESTIMATED VISCOSITY $\mu'$
AT HEMOGLOBIN DENSITY $Q_1$

$\mu' = Q_i (t_2 - t_1)$

PERFORM THIS ESTIMATION SEVERAL TIMES
IN A SIMILAR MANNER WHILE CHANGING $Q_1$,
AND GRAPH RELATIONSHIP OF BLOOD VISCOSITY
TO HEMOGLOBIN DENSITY

## Fig. 15

# Fig. 16

## CHANGE OVER TIME OF LIGHT RECEPTION INTENSITY

PRESSURE APPLIED
FOR 10 SECONDS

(A) EXAMPLE OF MEASUREMENT DATA

EXTRACT PRESSURE APPLICATION PORTION ONLY,
AND ANALYZE HEMOGLOBIN DENSITY

## CHANGE OVER TIME OF HEMOGLOBIN DENSITY

(B) EXAMPLE OF CHANGE OVER TIME
OF HEMOGLOBIN DENSITY

# Fig. 17

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11089808 A

- EP 1623668 A